# EUROPÄISCHE PATENTSCHRIFT

(11) **EP 1 280 778 B1**
(45) Veröffentlichungstag und Bekanntmachung des Hinweises auf die Patenterteilung: **02.08.2006**
(21) Anmeldenummer: 01964674.4
(22) Anmeldetag: 17.03.2001
(51) Int. Cl.: C07D 231/20, A01N 43/56

(54) **BENZOYLPYRAZOLE UND IHRE VERWENDUNG ALS HERBIZIDE**
BENZOYLPYRAZOLS AND THEIR USE AS HERBICIDES
PYRAZOLS DE BENZOYLE ET LEUR UTILISATION COMME HERBICIDES

(30) Priorität: 31.03.2000 DE 10016116
(43) Veröffentlichungstag der Anmeldung: 05.02.2003
(73) Patentinhaber: Bayer CropScience GmbH, 65929 Frankfurt (DE)
(72) Erfinder: SCHMITT, Monika, 60489 Frankfurt (DE); VAN ALMSICK, Andreas, 61440 Oberursel (DE); PREUSS, Rainer, 65510 Idstein (DE); WILLMS, Lothar, 65719 Hofheim (DE); AULER, Thomas, 65812 Bad Soden (DE); BIERINGER, Hermann, 65817 Eppstein (DE); THÜRWÄCHTER, Felix, 61348 Bad Homburg (DE)
(86) Internationale Anmeldenummer: PCT/EP2001/003070
(87) Internationale Veröffentlichungsnummer: WO 2001/074785

(56) Entgegenhaltungen:
- EP-A- 0 203 428
- DE-A- 2 513 750

## Beschreibung

Die Erfindung betrifft das technische Gebiet der Herbizide, insbesondere das der Herbizide zur selektiven Bekämpfung von Unkräutern und Ungräsem in Nutzpflanzenkulturen.

Aus verschiedenen Schriften ist bereits bekannt, daß bestimmte Benzoylpyrazole herbizide Eigenschaften besitzen. So sind in der deutschen Offenlegungsschrift DOS 25 13 750 solche 1-Alkyl-4-benzoyl-5-hydroxypyrazole und 1-Alkyl-4-benzoyl-5-thioypyrazole beschrieben, die am Phenylring vorzugsweise durch einen oder zwei Reste substituiert sind. Neben Wasserstoff werden als bevorzugte Reste in 2-Position Brom, Chlor, Jod, Methyl und Nitro, in 3-Position Methoxy, in 4-Position Chlor, Methoxy, Methylsulfonyl und Nitro sowie in 5 Position Methyl genannt. Die Hydroxy- beziehungsweise Thiogruppe der dort beschriebenen Verbindungen ist gegebenenfalls durch verschiedene Reste, wie Acylreste, substituiert. In J5 50533-454 werden weitere 5-Hydrdxypyrazole und 5-Thioypyrazole erwähnt, in denen die Hydroxy- beziehungsweise Thiogruppe grundsätzlich durch unterschiedliche Reste substituiert ist. US 4,643,757 offenbart 1-Methyl-4-benzoylpyrazole als Herbizide, die vorzugs-weise in 2-Position des Phenylrings Halogen, Nitro oder Sulfonylmethyl, in 3-Position Wasserstoff, Halogen oder Methyl und in 4-Position Halogen oder Sulfonylmethyl tragen. In EP-A 0 203 428 werden 1-Alkyl-4-benzoylpyrazole als Herbizide offenbart, die vorzugsweise in 2-Position des Phenylrings Halogen oder Methyl, in 3-Position Wasserstoff oder Methyl und in 4-Position Halogen oder Sulfonylmethyl tragen.

Die aus diesen Schriften bekannten Verbindungen zeigen jedoch häufig eine nicht ausreichende herbizide Wirksamkeit und/oder nicht ausreichende Verträglichkeit gegenüber Kulturpflanzen. Aufgabe der vorliegenden Erfindung ist daher die Bereitstellung von herbizid wirksamen Verbindungen mit - gegenüber den aus dem Stand der Technik bekannten Verbindungen - verbesserten herbiziden Eigenschaften sowie verbesserter Verträglichkeit gegenüber Kulturpflanzen.

Es wurde nun gefunden, daß bestimmte 4-Benzoylpyrazole, die durch ausgewählte Reste an speziellen Positionen substituiert sind, als Herbizide besonders gut geeignet sind. Ein Gegenstand der vorliegenden Erfindung sind daher Verbindungen der Formel (I) oder deren Salze 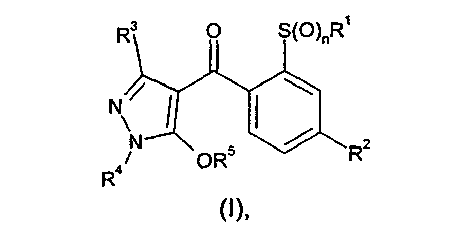 worin
- R¹: Methyl oder Ethyl;
- R²: Trifluormethyl;
- R³: Wasserstoff, Methyl oder Ethyl;
- R⁴: Methyl, Ethyl oder n-Propyl;
- R⁵: Wasserstoff, (C₁-C₆)-Alkylcarbonylmethyl, (C₁-C₄)-Alkylsulfonyl, Phenylsulfonyl, Benzyl, Benzoylmethyl, durch Halogen ein- oder mehrfach substituiertes (C₁-C₃)-Alkylsulfonyl, durch Methyl oder Halogen einfach substituiertes Phenylsulfonyl, durch Halogen, Nitro oder Methoxy substituiertes Benzyl oder durch Halogen, Nitro, Methyl oder Methoxy ein- oder mehrfach substituiertes Benzoylmethyl und
- n: 0, 1, oder 2 bedeuten.

Für den Fall, daß R⁵ Wasserstoff bedeutet, können die erfindungsgemäßen Verbindungen der Formel (I) in Abhängigkeit von äußeren Bedingungen, wie Lösungsmittel und pH-Wert, in unterschiedlichen tautomeren Strukturen auftreten: 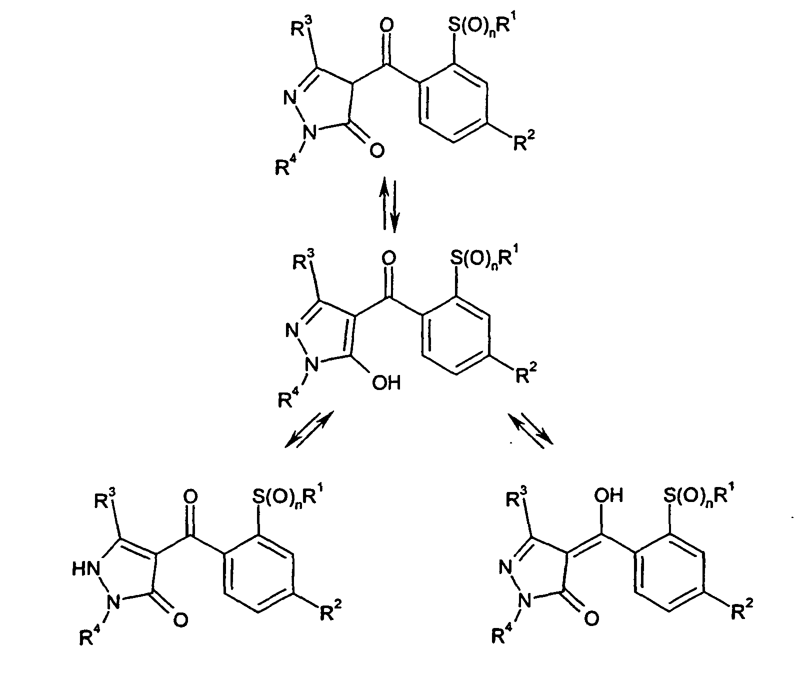 Je nach Art der Substituenten enthalten die Verbindungen der allgemeinen Formel (I) ein acides Proton, das durch Umsetzung mit einer Base entfernt werden kann. Als Basen eignen sich beispielsweise Hydride, Hydroxide und Carbonate von Lithium, Natrium, Kalium, Magnesium und Calcium sowie Ammoniak und organische Amine wie Triethylamin und Pyridin. Solche Salze sind ebenfalls Gegenstand der Erfindung.

In Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Halogen steht für Fluor, Chlor, Brom oder lod. Tosyl steht für 4-Methylphenylsulfonyl.

Ist eine Gruppe mehrfach durch Reste substituiert, so ist darunter zu verstehen, daß diese Gruppe durch ein oder mehrere gleiche oder verschiedene der genannten Reste substituiert ist.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

Von besonderem Interesse sind Verbindungen der allgemeinen Formel (I), worin n für 2 steht.

Bevorzugt sind Verbindungen der allgemeinen Formel (I), worin
- R¹: Methyl und
- R³: Wasserstoff oder Methyl bedeuten.

Bevorzugt sind auch Verbindungen der allgemeinen Formel (I), worin
- R⁴: Methyl oder Ethyl bedeutet.

Besonders bevorzugt sind Verbindungen der allgemeinen Formel (I), worin
- R⁵: Methylsulfonyl, Ethylsulfonyl, n-Propylsulfonyl, Phenylsulfonyl, 4-Methylphenylsulfonyl, Benzyl, Benzoylmethyl, Nitrobenzoylmethyl oder 4-Fluorbenzoylmethyl bedeutet.

Ebenfalls besonders bevorzugt sind Verbindungen der allgemeinen Formel (I), worin
- R⁵: Wasserstoff bedeutet.

Ganz besonders bevorzugt sind Verbindungen der allgemeinen Formel (I), worin
- R³: Methyl bedeutet.

In allen nachfolgend genannten Formeln haben die Substituenten und Symbole, sofern nicht anders definiert, dieselbe Bedeutung wie unter Formel (1) beschrieben.

Erfindungsgemäße Verbindungen, in denen R⁵ für Wasserstoff steht, können z.B. nach dem in Schema 1 angegebenen und aus DOS 25 13 750 bekannten Verfahren durch basenkatalysierte Umsetzung eines Benzoesäurehalogenids mit einem Pyrazolon oder gemäß dem in Schema 2 angegebenen und beispielsweise aus EP-A 0 186 117 bekannten Verfahren durch basenkatalysierte Umsetzung eines Benzoesäurehalogenids mit einem Pyrazolon und anschließender Umlagerung hergestellt werden. 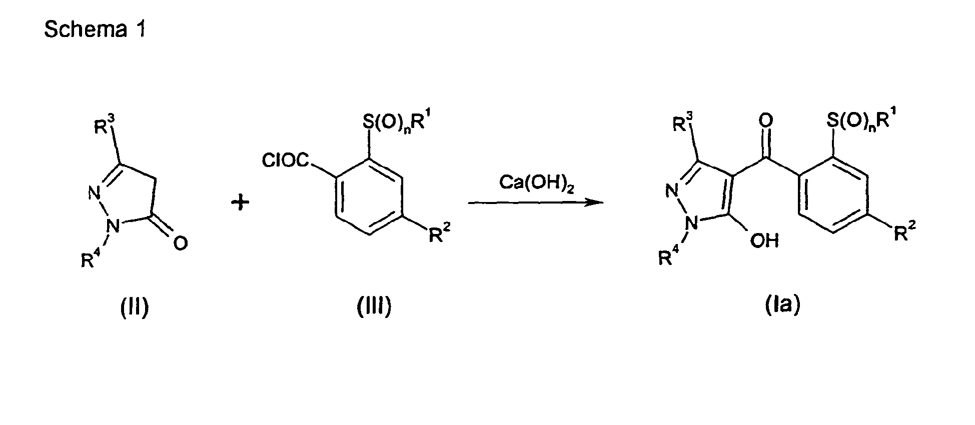 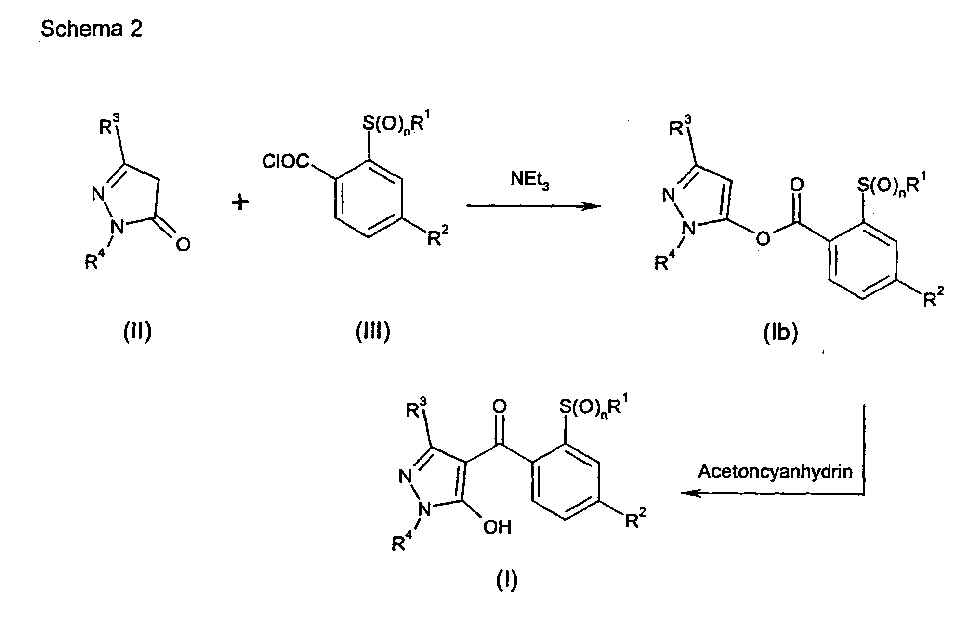 Erfindungsgemäße Verbindungen, in denen R⁵ eine andere Bedeutung als Wasserstoff hat, werden gemäß Schema 3 zweckmäßigerweise aus den nach Schema 1 oder 2 erhältlichen Verbindungen durch basenkatalysierte Reaktion mit einem geeigneten Acylierungsmittel R⁵-X, worin X für eine Abgangsgruppe wie Halogen steht, hergestellt. Solche Methoden sind beispielsweise aus DOS 25 13 750 bekannt. 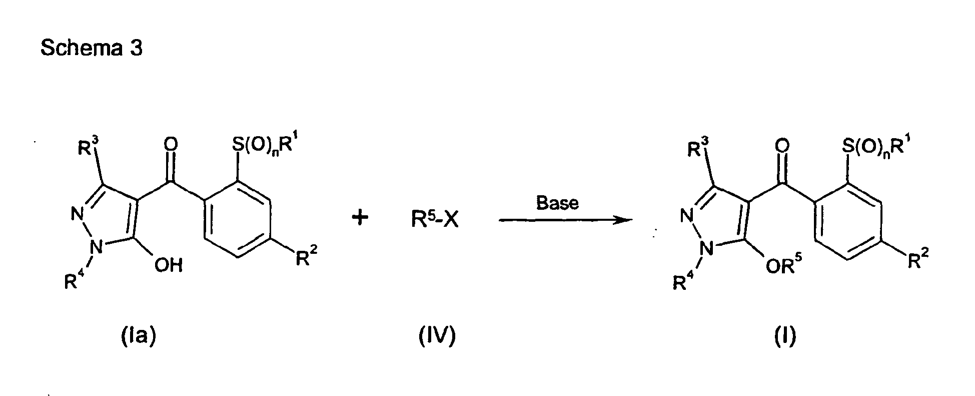 Die in obigen Schemata verwendeten Ausgangsverbindungen sind entweder käuflich oder nach an sich bekannten Methoden herstellbar. So können die Pyrazolone der Formel (II) beispielsweise nach den in EP-A 0 240 001 und J. Prakt. Chem. 315, 382, (1973) beschriebenen Methoden und die Benzoylchloride der Formel (III) nach den in EP-A 0 527 036 beschriebenen Verfahren hergestellt werden.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es in der Regel unerheblich, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll. Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauemde Cyperusarten gut erfaßt. Bei dikotylen Unkrautarten erstreckt sich das Wirkungsspektrum auf Arten wie z.B. Galium, Viola, Veronica, Lamium, Stellaria, Amaranthus, Sinapis, Ipomoea, Sida, Matricaria und Abutilon auf der annuellen Seite sowie Convolvulus, Cirsium, Rumex und Artemisia bei den perennierenden Unkräutern. Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft. Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird. Insbesondere zeigen die erfindungsgemäßen Verbindungen eine hervorragende Wirkung gegen Apera spica venti, Chenopodium album, Lamium purpureum, Polygonum convulvulus, Stellaria media, Veronica hederifolia, Veronica persica, Viola tricolor sowie gegen Arten von Amaranthus, Galium und Kochia.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Insbesondere weisen sie eine ausgezeichnete Verträglichkeit in Getreide, wie Weizen, Gerste und Mais, insbesondere Weizen, auf. Diese Verbindungen eignen sich daher sehr gut zur selektiven Bekämpfung von unerwünschtem Pflanzenwuchs in landwirtschaftlichen Nutzpflanzungen oder in Zierpflanzungen.

Aufgrund ihrer herbiziden Eigenschaften können diese Verbindungen auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregem von Pflanzenkrankheiten wie bestimmten Insekten oder Mikroorganismen wie Pilzen, Bakterien oder Viren. Andere besondere Eigenschaften betreffen z. B. das Emtegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Emteguts bekannt.

Bevorzugt ist die Anwendung der erfindungsgemäßen Verbindungen der Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (z.B. EP-A 0 242 236, EP-A 0 242 246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A 0 142 924, EP-A 0 193 259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431). Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfemt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann z.B. erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen. So sind transgene Pflanzen erhältlich, die veränderte Eigenschaften durch Über-expression, Suppression oder Inhibierung homologer (= natürlicher) Gene oder Gensequenzen oder Expression heterologer (= fremder) Gene oder Gensequenzen aufweisen.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen. Gegenstand der Erfindung ist deshalb auch die Verwendung der erfindungsgemäßen Verbindungen als Herbizide zur Bekämpfung von Schadpflanzen in transgenen Kulturpflanzen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Emteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Ein weiterer Gegenstand der Erfindung sind deshalb auch herbizide Mittel, die Verbindungen der Formel (I) enthalten. Die Verbindungen der Formel (I) können auf verschiedene Art formuliert werden, je nachdem welche biologischen und/oder chemisch-physikalischen Parameter vorgegeben sind. Als Formulierungsmöglichkeiten kommen z.B. in Frage: Spritzpulver (WP), wasserlösliche Pulver (SP), wasserlösliche Konzentrate, emulgierbare Konzentrate (EC), Emulsionen (EW), wie Öl-in-Wasser- und Wasserin-Öl-Emulsionen, versprühbare Lösungen, Suspensionskonzentrate (SC), Dispersionen auf ÖI- oder Wasserbasis, ölmischbare Lösungen, Stäubemittel (DP), Kapselsuspensionen (CS), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, 2,2'-dinaphthylmethan-6,6'-disulfonsaures Natrium, ligninsulfonsaures Natrium, dibutylnaphthalin-suffonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen fein gemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, DMF, Xylol oder auch höhersiedenden Aromaten oder Kohlenwasserstoffen oder Mischungen dieser Lösungsmittel unter Zusatz von einem oder mehreren Tensiden ionischer und/oder nichtionischer Art (Emulgatoren) hergestellt. Als Emulgatoren können z.B. verwendet werden: Alkylarylsulfonsaure Calzium-Salze wie Ca-dodecylbenzol-sulfonat oder nichtionische Emulgatoren wie Fettsäure-polyglykolester, Alkylaryl-polyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylen-sorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde. Suspensionskonzentrate können auf Wasser- oder Ölbasis sein. Sie können beispielsweise durch Naß-Vermahlung mittels handelsüblicher Perlrnühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührem, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden. Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischem und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57. Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.%, Wirkstoff der Formel (I). In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenem, Düngemitteln und/oder Wachstumsregulatoren herstellen, z.B. in Form einer Fertigformulierung oder als Tankmix.

Als Kombinationspartner für die erfindungsgemäßen Wirkstoffe in Mischungsformulierungen oder im Tank-Mix sind beispielsweise bekannte Wirkstoffe einsetzbar, wie sie z.B. in Weed Research 26, 441-445 (1986) oder "The Pesticide Manual", 11th edition, The British Crop Protection Council and the Royal Soc. of Chemistry, 1997 und dort zitierter Literatur beschrieben sind. Als bekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und - essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor, butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazapyr; imazamethabenz-methyl; imazaquin und Salze wie das Ammoniumsalz; ioxynil; imazethamethapyr; imazethapyr; imazosulfuron; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor, metham; methabenzthiazuron; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monolinuron; monuron; monocarbamide dihydrogensulfate; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor, primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und - methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1 H-1,2,4-triazol-1-carboxamid; thenylchlor (NSK-850); thiazafluron; thiazopyr (Mon-13200); thidiazimin (SN-24085); thiobencarb; thifensulfuron-methyl; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vemolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha, insbesonders zwischen 0,005 und 250 g/ha.

Die nachstehenden Beispiele erläutern die Erfindung.

### A. Chemische Beispiele

Die Herstellung der Ausgangsverbindungen 2-Methylsulfenyl-4-trifluormethylbenzoesäure, 2-Methylsulfinyl-4-trifluormethyl-benzoesäure und 2-Methylsulfonyl-4-trifluormethyl-benzoesäure erfolgte gemäß EP-A 0 527 036, die Herstellung von 5-Hydroxypyrazolen erfolgte gemäß EP-A 0 240 001 oder sind käuflich erhältlich.

### 1. Herstellung von 4-(4-Trifluormethyl-2-methylsulfonylbenzoyl)-5-hydroxy-1-ethyl-3-methylpyrazol

Schritt 1: 1-Ethyl-3-methyl-5-pyrazolyl-4-trifluormethyl-2-methylsulfonylbenzoat 2.1 g (7.8 mmol) 2-Methylsulfonyl-4-trifluormethyl-benzoesäure wurden in 90 ml CH₂Cl₂ gelöst. 2 Tropfen DMF und 2.98 (2.4 mmol) (COCl)₂ wurden hinzugefügt und 4 h unter Rückfluß gekocht. Dann wurde eingeengt, und der Rückstand in 300 ml CH₂Cl₂ aufgenommen und bei 0 °C mit 1.46g (9 mmol) 1-Ethyl-3-methyl-5-hydroxypyrazol und 4.45 ml NEt₃ versetzt. Man ließ 4 h bei Raumtemperatur nachrühren. Danach wurde eingeengt und chromatographisch gereinigt (Kieselgel, Essigester:Hexan = 3:2). Man erhielt 1-Ethyl-3-methyl-5-pyrazolyl-4-trifluormethyl-2-methylsulfonylbenzoat als Feststoff.
Ausbeute: 2.7 g (95 % der Theorie) R_{f} (Essigester): 0.75
¹H-NMR: δ [CDCl₃] 1.42 (t, 3H), 2.25 (s, 3H), 3.25 (s, 3H), 4.05 (q, 2H), 6.08 (s, 1H), 7.45 (d, 1 H), 7.65 (s, 1 H), 8.24 (d, 1 H).

### Schritt 2: 4-(4-Trifluormethyl-2-methylsulfonylbenzoyl}-5-hydroxy-1-ethyl-3-methylpyrazol

1.27 g (3.4 mmol) 1-Ethyl-3-methyl-5-pyrazolyl-4-trifluormethyl-2-methylsulfonylbenzoat, 2 Tropfen Acetoncyanhydrin und 0.8 ml (5.8 mmol) NEt₃ wurden in 80 ml CH₃CN gelöst und über Nacht bei Raumtemperatur gerührt. Dann wurde vollständig eingeengt, mit Wasser versetzt und mit 2 N HCl sauer gestellt. Das ausgefallene Produkt wurde abgesaugt und aus Ethanol umkristallisiert. Man erhielt 4-(4-Trifluormethyl-2-methylsulfonylbenzoyl)-5-hydroxy-1-etyhl-3methylpyrazol als gelbliches Öl. Ausbeute: 1.22 g (96 % der Theorie)
¹H-NMR: δ [CDCl₃] 1.45 (t, 3H), 2.25 (s, 3H), 2.95 (s, 3H), 4.00 (q, 2H), 7.65 (d, 1H), 7.85 (d, 1 H), 8.58 (s, 1 H).

### 2. Herstellung von 4-(4-Trifluormethyl-2-methylsulfonylbenzoyl)-1-ethyl-3-methyl-5-pyrazolyl-tosylat

0.37 g (1 mmol) 4-(4-Trifluormethyl-2-methylsulfonylbenzoyl)-5-hydroxy-1-etyhl-3-methylpyrazol und 0.20 g (1.1 mmol) p-Tos-Cl wurden in 20 ml CH₃CN gelöst. Dann wurden 0.26 g (1.8 mmol) Kaliumcarbonat hinzugefügt und 12 h bei Raumtemperatur gerührt. Es wurde mit Wasser verdünnt und mit Essigsester extrahiert. Es wurde mit MgSO₄ getrocknet und eingeengt. Man erhielt 4-(4-Trifluormethyl-2-methylsulfonylbenzoyl)-1-ethyl-3-methyl-5-pyrazolyltosylat als Wachs.
Ausbeute: 0.51 g (98 % der Theorie)
¹H-NMR: δ[CDCl₃] 1.90 (t, 3H), 2.05 (s, 3H), 2.45 (s, 3H), 3.25 (s,3H), 4.05 (q, 2H), 7.35 (d, 2H), 7.45 (d, 1H), 7.75 (d, 2H), 8.05 (d, 1 H), 8.40 (s, 1H).

Die in nachfolgenden Tabellen aufgeführten Beispiele wurden analog oben genannten Methoden hergestellt beziehungsweise sind analog oben genannten Methoden erhältlich.

Die verwendeten Abkürzungen bedeuten:

| | | | |
|---|---|---|---|
| Bn = Benzyl | Bz = Benzoyl | Et = Ethyl | Me = Methyl |
| Pr = Propyl | Ph = Phenyl | Tos = Tosyl | Fp. = Festpunkt |

Tabelle A: Erfindungsgemäße Verbindungen der allgemeinen Formel (I), worin die Substituenten und Symbole folgende Bedeutungen haben:

| | | |
|---|---|---|
| R¹ = Me | R² = CF₃ | n = 2 |

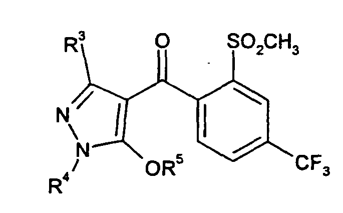

| **Nr.** | **R**^{**3**} | **R**^{**4**} | **R**^{**5**} | **Physikalische Daten** |
|---|---|---|---|---|
| 1 | Me | Et | H | ¹H-NMR-Daten siehe Herstellbeispiel 1 |
| 2 | Me | Et | Tos | ¹H-NMR-Daten siehe Herstellbeispiel 2 |
| 3 | Me | Et | Bz-CH₂ | |
| 4 | Me | Me | H | Fp. 202-204 °C |
| 5 | Me | Me | 4-F-Bz-CH₂ | Öl |
| 6 | Me | Me | Ph-SO₂ | |
| 7 | Me | Me | Bz-CH₂ | Öl |
| 8 | Me | Me | 4-NO₂-Bz-CH₂ | Öl |
| 9 | Me | Me | 3-NO₂Bz-CH₂ | Öl |
| 10 | Me | Me | Tos | Fp. 130-132 °C |
| 11 | Me | Me | n-Pr-SO₂ | Wachs |
| 12 | Me | Me | Bn | Fp. 179 °C |
| 13 | Me | Me | Me-SO₂ | Fp. 147°C |
| 14 | Me | Me | 2-NO₂-Bn | Fp. 146 °C |
| 15 | Me | Et | Me-SO₂ | Öl |
| 16 | Me | Et | Me-SO₂ | Öl |
| 17 | Me | Et | Bn | glasartig |
| 18 | Me | Et | 4-F-Bz-CH₂ | |

### B. Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man

| | | |
|---|---|---|
| 75 | Gew.-Teile einer | Verbindung der allgemeinen Formel(I), |
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man

| | | |
|---|---|---|
| 25 | Gew.-Teile einer | Verbindung der allgemeinen Formel (I), |
| 5 | " | 2,2'-dinaphthylmethan-6,6'-disulfonsaures Natrium, |
| 2 | " | oleoylmethyltaurinsaures Natrium, |
| 1 | " | Polyvinylalkohol, |
| 17 | " | Calciumcarbonat und |
| 50 | " | Wasser |

auf einer Kolloidmühle homogenesiert und vorzerkleinert, anschließend auf einer Perlmühte mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 l/ha in einer in den Tabellen 1 bis 5 angegebenen Dosierung auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der Verbindungen im Vergleich zu Verbindungen, die im Stand der Technik offenbart sind, bonitiert. Wie die Ergebnisse der Vergleichstabellen 1 bis 4 zeigen, weisen die ausgewählten erfindungsgemäßen Verbindungen dabei eine besssere herbizide Wirksamkeit gegen ein breites Spektrum wirtschaftlich wichtiger mono- und dikotyler Schadpflanzen auf als die im Stand der Technik offenbarten.

### 2. Kulturpflanzenverträglichkeit

In weiteren Versuchen im Gewächshaus werden Samen von Gerste und mono- und dikotyler Schadpflanzen in sandigem Lehmboden ausgelegt, mit Erde abgedeckt und im Gewächshaus aufgestellt, bis die Pflanzen zwei bis drei echte Blätter entwickelt haben. Die Behandlung mit den erfindungsgemäßen Verbindungen der Formel (I) und im Vergleich dazu mit den im Stand der Technik offenbarten erfolgt dann wie oben unter Punkt 1 beschrieben. Vier bis fünf Wochen nach der Applikation und Standzeit im Gewächshaus wird mittels optischer Bonitur festgestellt, daß die erfindungsgemäßen Verbindungen im Gegensatz zu den im Stand der Technik offenbarten Verbindungen die Kulturpflanze selbst bei höheren Wirkstoffdosierungen ungeschädigt lassen (s. Tabelle 5).

In den Vergleichsversuchen verwendete und im Stand der Technik offenbarte Verbindungen

| Nr | Struktur |
|---|---|
| S1 | 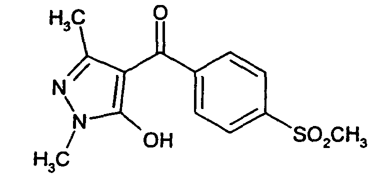 |
| S2 | 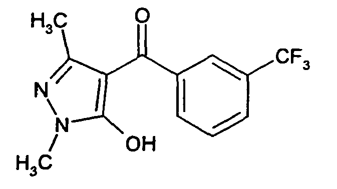 |
| S3 | 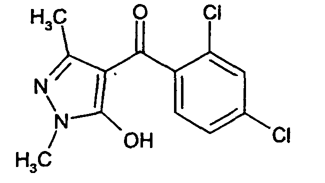 |

Die in folgenden Vergleichstabellen verwendeten Abkürzungen bedeuten:

| | | | |
|---|---|---|---|
| APSEV | Apera spica venti | CHEAL | Chenopodium album |
| LAMPU | Lamium purpureum | POLCO | Polygonum convolvulus |
| STEME | Stellaria media | VERHE | Veronica hederifolia |
| VERPE | Veronica persica | VIOTR | Viola tricolor |
| HORVS | Hordeum vulgaris | | |

Vergleichstabelle 1

| Verbindung Nr. | Dosierung [g.a.i./ha] | Schädigung der Schadpflanzen in % | | |
|---|---|---|---|---|
| | | POLCO | VERHE | VIOTR |
| 4 aus Tabelle A | 200 | 90 | 90 | 80 |
| S1 | 200 | 20 | 30 | 30 |

Vergleichstabelle 2

| Verbindung Nr. | Dosierung [g a.i./ha] | Schädigung der Schadpflanzen in % | | |
|---|---|---|---|---|
| | | CHEAL | POLCO | STEME |
| 10 aus Tabelle A | 50 | 95 | 60 | 70 |
| S2 | 50 | 0 | 10 | 10 |

Vergleichstabelle 3

| Verbindung Nr. | Dosierung [g a.i./ha] | Schädigung der Schadpflanzen in % | |
|---|---|---|---|
| | | CHEAL | STEME |
| 4 aus Tabelle A | 100 | 90 | 85 |
| S2 | 100 | 0 | 10 |

Vergleichstabelle 4

| Verbindung Nr. | Dosierung [g a.i./ha] | Schädigung der Schadpflanzen in % | | |
|---|---|---|---|---|
| | | LAMPU | VERHE | VERPE |
| 8 aus Tabelle A | 50 | 70 | 60 | 100 |
| S3 | 50 | 20 | 10 | 10 |

Vergleichstabelle 5

| Verbindung Nr. | Dosierung [g a.i/ha | Schädigung der Nutzpflanzen in % |
|---|---|---|
| | | HORVS |
| 1 aus Tabelle A | 200 | 0 |
| 4 aus Tabelle A | 200 | 0 |
| S2 | 200 | 20 |
| S3 | 200 | 20 |

## Patentansprüche

1. Benzoylpyrazole der Formel (I) oder deren Salze 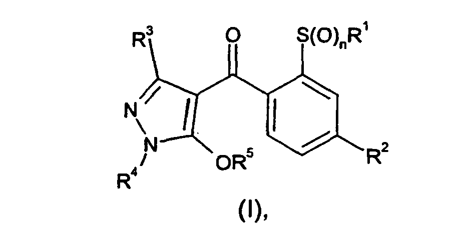 worin
R¹ Methyl oder Ethyl;
R² Trifluormethyl;
R³ Wasserstoff, Methyl oder Ethyl;
R⁴ Methyl, Ethyl oder n-Propyl;
R⁵ Wasserstoff, (C₁-C₆)-Alkylcarbonylmethyl, (C₁-C₄)-Alkylsulfonyl, Phenylsulfonyl, Benzyl, Benzoylmethyl, durch Halogen ein- oder mehrfach substituiertes (C₁-C₃)-Alkylsulfonyl, durch Methyl oder Halogen einfach substituiertes Phenylsulfonyl, durch Halogen, Nitro oder Methoxy substituiertes Benzyl oder durch Halogen, Nitro, Methyl oder Methoxy ein- oder mehrfach substituiertes Benzoylmethyl und
n 0, 1, oder 2 bedeuten.

2. Benzoylpyrazole nach Anspruch 1, worin
R¹ Methyl und
R³ Wasserstoff oder Methyl bedeuten.

3. Benzoylpyrazole nach einem der Ansprüche 1 oder 2, worin R⁴ Methyl oder Ethyl bedeutet.

4. Benzoylpyrazole nach einem der Ansprüche 1 bis 3, worin
R⁵ Wasserstoff, Methylsulfonyl, Ethylsulfonyl, n-Propylsulfonyl, Phenylsulfonyl, 4-Methylphenylsulfonyl, Benzyl, Benzoylmethyl, Nitrobenzoylmethyl oder 4-Fluorbenzoylmethyl bedeutet.

5. Benzoylpyrazole nach einem der Ansprüche 1 bis 4, worin R³ Methyl bedeutet.

6. Herbizide Mittel, **gekennzeichnet durch** einen herbizid wirksamen Gehalt an mindestens einer Verbindung der allgemeinen Formel (I) gemäß einem der Ansprüche 1 bis 5.

7. Herbizide Mittel nach Anspruch 6 in Mischung mit Formulierungshilfsmitteln.

8. Verfahren zur Bekämpfung unerwünschter Pflanzen, **dadurch gekennzeichnet, daß** man eine wirksame Menge mindestens einer Verbindung der allgemeinen Formel (I) nach einem der Ansprüche 1 bis 5 oder eines herbiziden Mittels nach Anspruch 6 oder 7 auf die Pflanzen oder auf den Ort des unerwünschten Pflanzenwachstums appliziert.

9. Verwendung von Verbindungen der allgemeinen Formel (I) nach einem der Ansprüche 1 bis 5 oder von herbiziden Mitteln nach Anspruch 6 oder 7 zur Bekämpfung unerwünschter Pflanzen.

10. Verwendung nach Anspruch 9, worin die Verbindungen der allgemeinen Formel (I) zur Bekämpfung unerwünschter Pflanzen in Kulturen von Nutzpflanzen eingesetzt werden.

11. Verwendung nach Anspruch 10, worin die Nutzpflanzen transgene Nutzpflanzen sind.

## Claims

1. A benzoylpyrazole of the formula (I) or a salt thereof 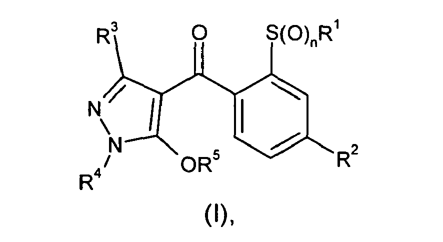 in which
R¹ is methyl or ethyl;
R² is trifluoromethyl;
R³ is hydrogen, methyl or ethyl;
R⁴ is methyl, ethyl or n-propyl;
R⁵ is hydrogen, (C₁-C₆)-alkylcarbonylmethyl, (C₁-C₄)-alkylsulfonyl, phenylsulfonyl, benzyl, benzoylmethyl, (C₁-C₃)-alkylsulfonyl which is mono- or polysubstituted by halogen, phenylsulfonyl which is monosubstituted by methyl or halogen, benzyl which is substituted by halogen, nitro, methyl or methoxy or benzoylmethyl which is mono- or polysubstituted by halogen, nitro, methyl or methoxy and
n is 0,1, or 2.

2. A benzoylpyrazole as claimed in claim 1, in which
R¹ is methyl and
R³ is hydrogen or methyl.

3. A benzoylpyrazole as claimed in claim 1 or 2, in which R⁴ is methyl or ethyl.

4. A benzoylpyrazole as claimed in any of claims 1 to 3, in which
R⁵ is hydrogen, methylsulfonyl, ethylsulfonyl, n-propylsulfonyl, phenylsulfonyl, 4-methylphenylsulfonyl, benzyl, benzoylmethyl, nitrobenzoylmethyl or 4-fluorobenzoylmethyl.

5. A benzoylpyrazole as claimed in any of claims 1 to 4, in which R³ is methyl.

6. A herbicidal composition which comprises a herbicidally effective amount of at least one compound of the formula (I) as claimed in any of claims 1 to 5.

7. The herbicidal composition as claimed in claim 6 in a mixture with formulation auxiliaries.

8. A method for controlling undesirable plants, which comprises applying an effective amount of at least one compound of the formula (I) as claimed in any of claims 1 to 5 or a herbicidal composition as claimed in claim 6 or 7 on to the plants or the location of the undesirable vegetation.

9. The use of compounds of the formula (I) as claimed in any of claims 1 to 5 or of herbicidal compositions as claimed in claim 6 or 7 for controlling undesirable plants.

10. The use as claimed in claim 9, wherein the compounds of the formula (I) are used for controlling undesirable plants in crops of useful plants.

11. The use as claimed in claim 10, wherein the useful plants are transgenic useful plants.

## Revendications

1. Benzoylpyrazoles de formule (I) ou leurs sels 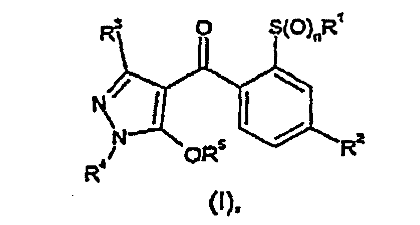 dans laquelle
R¹ représente un groupe méthyle ou éthyle ;
R² représente un groupe trifluorométhyle ;
R³ représente un atome d'hydrogène, un groupe méthyle ou éthyle ;
R⁴ représente un groupe méthyle, éthyle ou n-propyle ;
R⁵ représente un atome d'hydrogène, un groupe (alkyl en C₁-C₆) carbonyméthyle, (alkyl en C₁-C₄)-sulfonyle, phénylsulfonyle, benzyle, benzoylméthyle, (alkyl en C₁-C₃)sulfonyle substitué une ou plusieurs fois par un substituant halogène, phénylsulfonyle substitué une fois par un substituant méthyle ou halogène, benzyle substitué par un substituant halogène, nitro ou méthoxy, ou benzoylméthyle substitué une ou plusieurs fois par un substituant halogène, nitro, méthyle ou méthoxy, et
n vaut 0, 1 ou 2.

2. Benzoylpyrazoles selon la revendication 1, où
R¹ représente un groupe méthyle et
R³ représente un atome d'hydrogène ou un groupe méthyle.

3. Benzoylpyrazoles selon l'une des revendications 1 ou 2, où R⁴ représente un groupe méthyle ou éthyle.

4. Benzoylpyrazoles selon l'une des revendications 1 à 3, où
R⁵ représente un atome d'hydrogène, un groupe méthylsulfonyle, éthylsulfonyle, n-propylsulfonyle, phénylsulfonyle, 4-méthylphénylsulfonyle, benzyle, benzoylméthyle, nitrobenzoylméthyle ou 4-fluorobenzoylméthyle.

5. Benzoylpyrazoles selon l'une des revendications 1 à 4, où R³ représente un groupe méthyle.

6. Agent herbicide **caractérisé par** une teneur à efficacité herbicide en au moins un composé de formule générale (I) selon l'une des revendications 1 à 5.

7. Agent herbicide selon la revendication 9, en mélange avec des formulants.

8. Procédé pour la lutte contre des plantes indésirables, **caractérisé en ce qu'**on applique une quantité efficace d'au moins un composé de formule générale (I) selon l'une des revendications 1 à 5 ou d'un agent herbicide selon la revendication 6 ou 7, aux plantes ou à l'endroit de la croissance végétale indésirable.

9. Utilisation de composés de formule générale (I) selon l'une des revendications 1 à 5 ou d'agents herbicides selon la revendication 6 ou 7 dans la lutte contre une végétation indésirable.

10. Utilisation selon la revendication 9, dans laquelle on utilise les composés de formule générale (I) dans la lutte contre des plantes indésirables dans des cultures de plantes utiles.

11. Utilisation selon la revendication 10, dans laquelle les plantes utiles sont des plantes transgéniques utiles.
